# EUROPEAN PATENT APPLICATION

(11) **EP 2 489 270 A1**
(43) Date of publication of application: **22.08.2012**
(21) Application number: 10818759.2
(22) Date of filing: 17.09.2010
(51) Int. Cl.: A01N 63/02, A01G 7/06, A01P 3/00, C12N 1/20, C12R 1/01

(54) **PLANT-REARING AGENT, PLANT DISEASE RESISTANCE INDUCER, AND PLANT DISEASE CONTROL METHOD**

(30) Priority: 28.09.2009 JP 2009222962
(71) Applicant: Soma, Genichiro, Setagaya-ku Tokyo 158-0084 (JP); Biomedical Research Group Inc., Tokyo 158-0084 (JP); Macrophi, Inc., Takamatsu-shi, Kagawa 761-0301 (JP)
(72) Inventor: NAKATA Yohko, Mihara-shi Hiroshima 729-0413 (JP); KOHCHI Chie, Takamatsu-shi Kagawa 761-8075 (JP); INAGAWA Hiroyuki, Takamatsu-shi Kagawa 761-8062 (JP); GOMI Kenji, Yokohama-shi Kanagawa 245-0015 (JP); SOMA, Genichiro, Tokyo 158-0084 (JP)
(74) Representative: Appelt, Christian W.
(86) International application number: PCT/JP2010/066202
(87) International publication number: WO 2011/037086

(57) **Abstract**

A disease resistance capacity of vegetables, grasses and flowers, etc. , is enhanced and their survival rates are improved by growing plants in an appropriate coexistence of a glycolipid derived from Pantoea agglomerans or a composition containing the glycolipid. Thereby, a method of augmenting disease resistance of vegetables, grasses and flowers, etc. , and growing agricultural crops with high quality is provided, and further a novel plant growing agent and plant disease resistance inducer that enables this are provided. As a result, it becomes possible to cultivate agricultural crops or increase productivity with as little use of agricultural chemicals as possible. Even if an amount of the agricultural chemical is reduced, a cultivation success rate of agricultural crops can be enhanced and immunity of the plant body can be enhanced even where useful soil bacteria are not frequently present. Thus, new types of agricultural crop businesses such as contract cultivations by means of organic cultivation, agricultural chemical-free cultivation and hydroponic cultivation are promoted.

## Description

### [Technical Field]

The present invention relates to a plant growing agent, a plant disease resistance inducer, and a plant disease control method, which improve survival rates by enhancing the innate immunity function of plants using natural products to prevent transmission of diseases, and are effective for high yields of agricultural crops and horticultural crops and production of crops with high quality.

### [Background Art]

"Safe and secure agricultural crops" are strongly desired by consumers. In response to these needs, growing methods using reduced or no agricultural chemicals, such as growing organic vegetables (crops) has been recommended in recent years at production sites of agricultural crops. However, under actual circumstances the use of chemicals is required in a similar manner as is conventionally done for the occurrence of diseases and chemical fertilizers are still used for improving productivity at production sites. Therefore, a growing method that is for controlling the occurrence of disease as well as promoting the growth of agricultural crops and is easily used, is safe, secure and effective at production fields, or a novel plant growth accelerator that enables this has been demanded. That is, it is necessary to achieve the purpose by a novel mechanism that is different from conventional plant growth acceleration and disease control. The present inventors researched extensively and carried out developments concerning this purpose, and developed a safe, secure and effective growing method by a novel mechanism and a novel plant growth accelerator that enables this, and completed the present invention.

The original point of the present invention is to focus on the innate immune effect that is a defense mechanism against infection, which a plant itself has intrinsically. In a narrow sense, an immune mechanism is recognized as a function inherent to organisms for protecting an individual from transmission of an infectious agent. And, at least two types, that is, an acquired immune system and an innate immune system are known as immune mechanisms. An acquired immunity is described as being an immune mechanism inherent to vertebrates, and cells that serve the primary role are lymphocytes belonging to white blood cells. On the other hand, an innate immunity is described as being present in all living animals, and cells that serve the primary role are phagocytes (for example, macrophages) characterized by phagocytozing foreign material.

That is, the innate immunity covers most infection control effects in animals. In fact, the present inventors have shown that as well as achieving infection control, survival rates are also improved in cultured marine and domestic animals using methods of activating innate immunity (Non-Patent Literature 1). Meanwhile in plants, static resistance and dynamic resistance are observed as an action similar to the innate immune mechanism in animals, but there is no evidence that the phagocytes are present in plants. However, according to known literature, as reported by a group from a public study research institute in Spain, effects of living Pantoea spray on molds in preserved apples in 2001 in the case of gram-negative bacteria increased infection resistance against plant pathogens. In this public study research institute, living Pantoea is sold as a bio-control agent (Non-Patent Literature 2). Further, more recently, it has been disclosed that glycolipids derived from Xanthomonas and Ralstonia are plant pathogens that induce infection control in plants (Non-Patent Literatures 3 and 4). That is, it has been disclosed that infection control against pathogens is induced by treating the plant with glycolipids derived from pathogens. This suggests that there is an innate immune mechanism in plants as with animals.

Moreover, the present inventors discovered in 1992 that glycolipids derived from Pantoea agglomerans living in a symbiotic relationship with wheat enhanced orally or transdermally the infection resistance in animals and humans and had an ameliorating effect on various diseases (Non-Patent Literature 5). This is because glycolipids activate the innate immunity function including phagocytes via mucosa in animals and humans. Thus, the present inventors considered that innate immunity including phagocytes were also present in plants and that the survival rates of plants were improved and infection resistance was augmented by activating the innate immune mechanism. According to conventional technology, the induction of infection control in plants is characterized by using glycolipids derived from plant and animal pathogens. This idea is a method based on a similar perspective as the infection control method currently being studied in aquaculture, and is referred to as a vaccine in a broad sense. However, according to the idea of the present inventors, if the innate immune mechanism is present in plants, the glycolipids used for the activation of the innate immune mechanism are not necessarily derived from plant pathogens, and may be derived from any microorganism as long as the innate immune mechanism of the plant is optimally activated. Therefore, the glycolipids derived from edible microorganisms used for fermentation, or the like and microorganisms intrinsically living in a symbiotic relationship with plants are considered useful for improving survival rates and for infection control in plants. The glycolipids derived from microorganisms living in a symbiotic relationship with plants have been eaten by humans for a long time, and thus, it is not too much to say that the safety of glycolipids is guaranteed for animals and humans.

The present invention has been completed by finding that glycolipids derived from Pantoea agglomerans that are not plant pathogens have an infection control effect in plants. More specifically, it was shown that glycolipids derived from Pantoea agglomerans were applied to infected rice with bacterial leaf blight and glycolipids derived from Pantoea agglomerans inhibited rice blight. From this, it is obvious that glycolipids derived from Pantoea agglomerans enhance the infection resistance of plants irrespective of pathogenicity against plants. In addition, the glycolipids derived from microorganisms living in a symbiotic relationship with or parasitizing the plant or microorganisms used for fermentation, or the like have been eaten by humans. Thus, the use of these glycolipids has led to the development of a novel technology that improvements in infection control and improvements in survival rates are achieved by utilizing the innate immune mechanism that plants intrinsically have in the need for safe and secure foods. Further, this novel technology clearly contributes to the solution of environmental problems by reducing the amount of agricultural chemicals to be used.

### [Citation List]

### [Patent Literature]

[Patent Literature 1] JP 4026722 B2
   [Non-Patent Literature]

[Non-Patent Literature 1] Y. Takahashi, M. Kondo, T. Itami, T. Honda, H. Inagawa, T. Nishizawa, G-I. Soma, Y. Yokomizo; Fish & Shellfish Immunology, 10 555-558 (2000)
[Non-Patent Literature 2] C. Nunes, J. Usall, N. Teixido, and I. Vinas; International Journal of Food Microbiology Vol. 70 53-61 (2001)
[Non-Patent Literature 3] N. Espositoa, O. G. Ovchinnikovab, A. Baronea, A. Zoinad, O. Holstb, and A. Evidente; CHEMISTRY & BIODIVERSITY Vol.5 2662-2675 (2008)
[Non-Patent Literature 4] Prime-A-Plant Group; MPMI Vol.19 1062-1071 (2006)
[Non-Patent Literature 5] D. Mizuno and G-I Soma; Molecular Biotherapy Vol.4 166-169 (1992)

### [Summary of Invention]

### [Technical Problem]

It is an object of the present invention to provide a method for achieving the improvement of survival rates of plants in a safe and secure manner as well as augmenting infection resistance by activating an innate immune mechanism that is present in plants, and a novel plant growing agent and plant disease resistance inducer that enables this.

### [Solution to Problem]

The plant growing agent of the present invention is characterized by containing a glycolipid derived from Pantoea agglomerans as an active ingredient.

The plant disease resistance inducer of the present invention is characterized by containing a glycolipid derived from Pantoea agglomerans as the active ingredient.

The plant growing agent of the present invention is also characterized in that a fermentation culture obtained by fermenting a plant with Pantoea agglomerans and culturing the Pantoea agglomerans is combined.

The plant disease resistance inducer of the present invention is also characterized in that the fermentation culture obtained by fermenting a plant with Pantoea agglomerans and culturing the Pantoea agglomerans is combined.

The disease control method of the present invention is characterized in that the disease in the plant is controlled by the above plant disease resistance inducer.

### [Advantageous Effects of the Invention]

According to the present invention, by enhancing the self defense function that a plant itself has, it becomes possible to enhance the resistance to pathogens, thereby cultivating agricultural crops and increasing productivity with as little use of agricultural chemicals as possible. Even when the amount of agricultural chemicals is reduced, the cultivation success rate of an agricultural crop is enhanced and the immunological capacity of the plant can be enhanced even where useful soil bacteria occur in smaller amounts. Thus, new types of crop businesses such as contract cultivation by means of organic cultivation, chemical-free cultivation, or hydroponic cultivation can be promoted.

### [Brief Description of Drawings]

[FIG. 1] FIG. 1 is a view (No. 1) showing the disease resistance to bacterial leaf blight of rice of the present invention;
[FIG. 2] FIG. 2 is a view (No. 2) showing the disease resistance to bacterial leaf blight of rice of the present invention;
[FIG. 3] FIG. 3 is a view (No. 3) showing the disease resistance to bacterial leaf blight of rice of the present invention; and
[FIG. 4] FIG. 4 is a view showing the disease resistance to strawberry powdery mildew.

### [Description of Embodiments]

Hereinafter, modes for carrying out the present invention are described in detail below with reference to the attached drawings.

### [Example 1]

### (Agent alone against bacterial leaf blight of rice)

A rice plant was grown to a fifth leaf stage. An edge of the fully extended fifth leaf was cut, and a cut surface was applied with a Pantoea agglomerans glycolipid diluted twice (Somacy-FL100, manufactured by MACROPHI Inc., a fermented plant extract that is a working product of the invention according to Patent Literature 1, that is, the fermented plant extract obtained by fermenting a wheat-amylase treatment liquid obtained by treating wheat with amylase with Pantoea agglomerans and simultaneously culturing Pantoea agglomerans). Distilled water was applied as a control. Pathogen of bacterial leaf blight of rice (Xanthomonas oryzae pv. oryzae) was inoculated (applied) immediately after (0h) and 6 hours (6 h) after treating with the glycolipid. The length of a lesion (length of the blight of the fifth leaf) was measured 10 days after the inoculation.

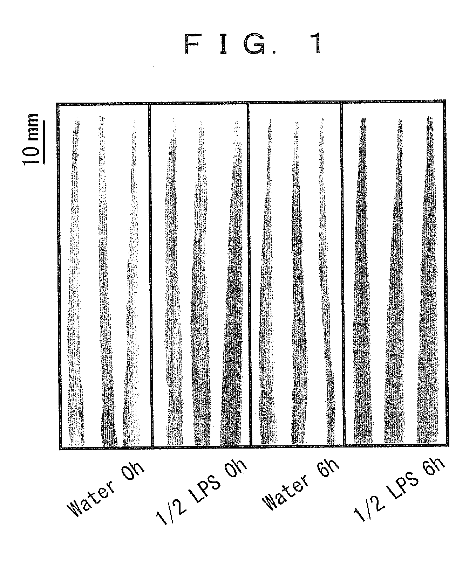

As a result, the leaf, the cut surface of which had been treated with distilled water was entirely withered with yellowing in both groups inoculated with the bacteria after 0 hours and after 6 hours. The leaf, the cut surface of which was treated with the glycolipid and the group that was inoculated with the bacteria after 0 hours was withered to the same extent as the leaf treated with the distilled water group, and the disease developed. The group inoculated with the pathogen after 6 hours with the glycolipid treatment kept a healthy color different from the other 3 groups, and a noticeable inhibition of disease development was observed (FIG. 1). "Water 0 h" denotes the group inoculated with the bacteria immediately after being treated with water, "1/2 LPS 0 h" denotes the group inoculated with the bacteria immediately after being treated with the glycolipid, "Water 6 h" denotes the group inoculated with the bacteria 6 hours after being treated with water, and "1/2 LPS 6 h" denotes the group inoculated with the bacteria 6 hours after being treated with the glycolipid.

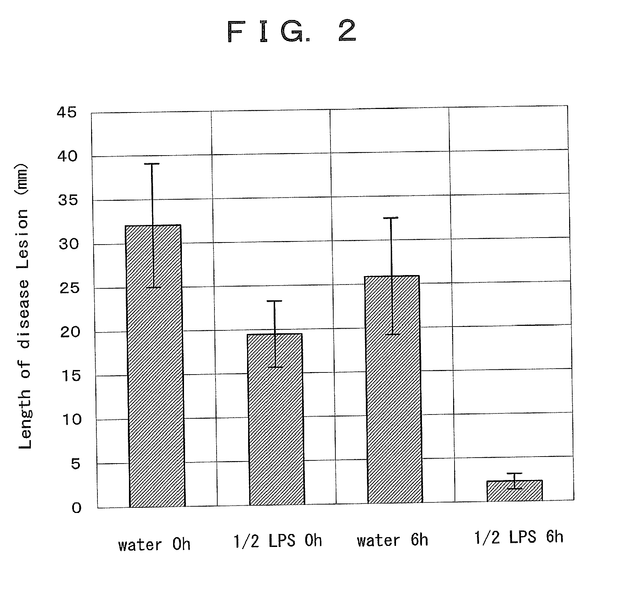

The length of the lesion in the group inoculated with the bacteria 6 hours after being treated with the glycolipid was 2.3 mm, which indicated that the development of the disease was statistically significantly inhibited to about 1/11 compared with 25. 5 mm in the group inoculated with the bacteria 6 hours after being treated with distilled water (one-way analysis of variance, Levene test P=0.0014) (FIG. 2). In the groups inoculated after 0 hours, the length of the lesion in the group treated with the glycolipid was 19.7 mm, which indicated that the development of the disease was statistically significantly inhibited to 2/3 or less compared with 31.9 mm in the group treated with distilled water (one-way analysis of variance, Levene test P=0.0016).

From this data, it became clear that the glycolipid is effective in disease resistance. Further, the effect was confirmed to be more noticeable when several hours passed after contacting the plant with the glycolipid.

### [Example 2]

### (Combination use with spreading agent against bacterial leaf blight of rice)

A rice plant was grown to a completely extended fifth leaf stage. A Pantoea agglomerans glycolipid (Somacy-FL100) diluted 10 times was mixed with a spreader (Bravo manufactured by AGRO-KANESHO Co., Ltd.) diluted 1, 000 times, which was then sprayed onto the entire rice plant mainly the fifth leaves. The spreading agent diluted 1,000 times alone was sprayed as the control. Pathogen of bacterial leaf blight of rice (Xanthomonas oryzae pv. oryzae) was inoculated in the fifth leaves 24 hours (24 h) after the treatment with the glycolipid. The length of the lesion (length of blight on the fifth leaf) was measured 14 days after the inoculation.

As a result, the leaf sprayed with the only spreader was entirely withered with yellowing. The leaves in the group treated with glycolipid containing the spreading agent and inoculated with the pathogen kept a healthy color different from the control group, and a noticeable inhibition of disease development was observed.

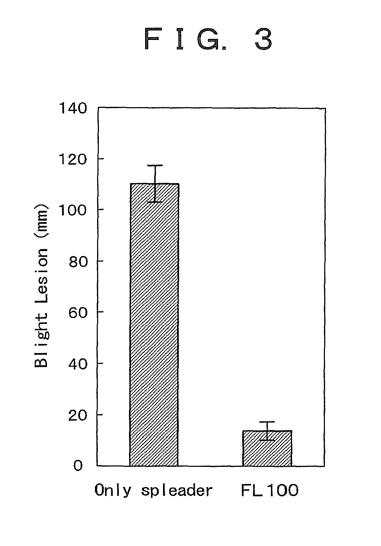

The length of the lesion in this group treated with the glycolipidwas 13.75 mm, which indicated that the development of the disease was statistically significantly inhibited to about 1/8 compared with 110. 58 mm in the control group (Student t test, P<0.05) (FIG. 3). "Only spreader" denotes the group sprayed with the only spreader and inoculated with the bacteria after 24 hours, and "FL100" denotes the group sprayed with the glycolipid mixed with the spreading agent and inoculated with the bacteria after 24 hours.
From this data, it became clear that the combination use treatment of the glycolipid with the spreader was effective for the induction of the disease resistance.

### [Example 3]

### (Strawberry anthracnose)

The strawberry used was controlled by leaf removal so that an extended leaf was in a fifth leaf stage. The Pantoea agglomerans glycolipid (Somacy-SL100, manufactured by MACROPHI Inc., a fermented plant extract that is a working product of the invention according to Patent Literature 1, which is for feedstuffs. Somacy-FL100 is for foods, which is obtained by removing impurities from Somacy-SL100 by filtration etc. Both are different in pH but the same in glycolipid content, which is 10 mg/mL) was adjusted to pH 7.0 to 7.5, and diluted with distilled water 100 times. A spreader (Agra, manufactured by AGRO-KANESHO Co., Ltd.) at 5000 times was added, and sufficiently sparged on seedlings in pots. As the comparison control, a Propineb hydrating agent (ANTRACOL granule hydrating agent manufactured by Bayer CropScience k. k.) at 500 times was sparged. Spores (1×10⁴/mL) of strawberry anthracnose fungus (Glomerella cingulata ED25 strain) were inoculated 6 hours after sparging the glycolipid. The plant was kept wet for 24 hours after the inoculation.
For an investigation into the onset of disease, the number of black spot lesions per leaf was observed 12 days after the sparging treatment, and an incidence of the disease (%) was calculated.

As a result, the incidence of the disease in the group sparged with the glycolipid was 16.6%, which was inferior to 0.7% in the group sparged with the agricultural chemicals in the inhibitory effect on the pathogenesis, but was about half compared with 30.2% in the group with no treatment. Thus, the inhibitory effect on the pathogenesis was observed (Table 1).
From this data, it became clear that the glycolipid is effective for the augmentation of the infection resistance to the strawberry anthracnose.

**[Table 1]**

| Treatment group | Incidence of disease (Number of lesions per plant) |
|---|---|
| Group sparged with glycolipid | 16.6% |
| Group sparged with agricultural chemical | 0.7% |
| Group with no treatment | 30.2% |
| Group with no inoculation | 0% |

### [Example 4]

### (Strawberry powdery mildew)

The strawberry was grown to an extended sixth leaf stage. A Pantoea agglomerans glycolipid (Somacy-SL100) was sparged onto fully extended plant. The glycolipid solution was adjusted to pH 7.0 to 7.5 and diluted with distilled water 20 times. A spreading agent at 1,000 times (Approach B1 manufactured by MARUWA Biochemical Co., Ltd.) was added, and was sparged on the front and the back of the leaves at 25 mL per plant. As the comparison controls, Trifmine hydrating agent diluted 3,000 times (manufactured by Nippon Soda Co., Ltd.) and a yeast extract diluted 1,000 times (manufactured by AGREVO Co. , Ltd.) were sparged. These solutions were sparged 5 times from February 23, 2010 at intervals of about 10 days, on March 5, March 15, March 25, and April 5. Pathogen was not inoculated.

An investigation into the onset of disease of strawberry powdery mildew was carried out by selecting four-leaves mainly newly extended leaves and measuring an extent of disease by a diseased area per leaflet, respectively. The investigation was performed on February 23, March 25, April 5, and April 15, and a rate of diseased leaflets and an incidence of disease were calculated. Drug-induced suffering was appropriately observed by the naked eye. The extent of disease is defined by a lesion area rate, and was scored as follows: 0 : no lesion, 1: <5%, 2: <25%, 3: <50%, and 4: >50%. The incidence of disease was calculated by Σ (Index by the extent of disease × Number of leaflets by the extent of disease) / (4 × Number of investigated leaflets) × 100.

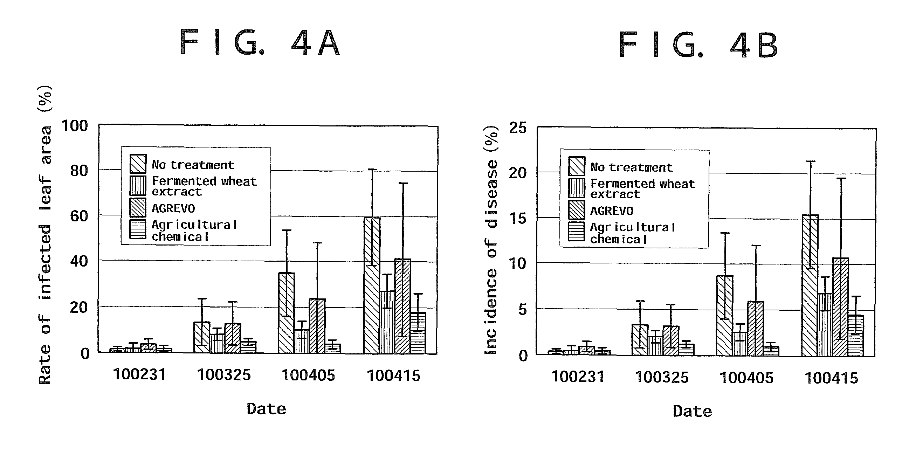

As a result, the rate of diseased leaflets was 10.4% on April 5 and 27.1% on April 15 in the group sparged with the glycolipid, 3.8% on April 5 and 17.7% on April 15 in the group treated with the agricultural chemicals, 24.0% on April 5 and 41.3% on April 15 in the group sparged with the yeast extract, and 34.8% on April 5 and 59.2% on April 15 in the group with no treatment (FIG. 4). In the figure, "No treatment" denotes the group with no treatment, "Fermented wheat extract" denotes the group sparged with the glycolipid, "AGREVO" denotes the group sparged with the yeast extract, "agricultural chemicals" denotes the group sparged with the agricultural chemical, "100231" denotes the investigation on February 23?, "100325" denotes the investigation on March 25, "100405" denotes the investigation on April 5, and "100415" denotes the investigation on April 15. Therefore, it was understood that although the group sparged with the glycolipid fell short of the treatment with the agricultural chemical, this inhibited the onset of disease compared with the group with no treatment. Further, it was shown that this inhibitory effect was higher than that of the yeast extract.

As for the incidence of disease, the group sparged with the glycolipid was observed not to have the same disease inhibitory ability as the group treated with the agricultural chemicals as was the case with the rate of diseased leaflets. However, the incidence of disease was 2.6 in the group sparged with the glycolipid and 8.7 in the group with no treatment on April 5, and 6.9 in the group sparged with the glycolipid and 15.5 in the group with no treatment on April 15. Thus, the incidence of disease in the group sparged with the glycolipid was half or less of that in the group with no treatment.
From this data, it became clear that the glycolipid was effective for augmenting infection resistance to strawberry powdery mildew.

The present invention is not limited to the above Examples.
When the plant disease resistance inducer of the present invention is used for the plant, its amount to be used is not particularly limited. However, the concentration of the glycolipid in Somacy-FL100 and Somacy-SL100 that are undiluted solutions of the working product of the present invention is 10 mg/mL. Thus, it is not realistic that a concentration higher than this concentration is used. On the other hand, when these undiluted solutions were diluted 200 times, disease resistance induction was hardly observed. Therefore, the concentration of the glycolipid is preferably 0.5 mg/mL or more and 10 mg/mL or less.

The disclosure of Japanese Patent Application No. 2009-222962, filed on September 28, 2009 including the specification, the claims and drawings, is incorporated herein by reference in its entirety.
All the publications, patents and patent applications cited in the present specification are incorporated in the present specification by reference in their entirety.

## Claims

1. A plant growing agent containing a glycolipid derived from Pantoea agglomerans as an active ingredient.

2. A plant disease resistance inducer containing a glycolipid derived from Pantoea agglomerans as an active ingredient.

3. A plant growing agent, wherein a fermented culture obtained by fermenting a plant with Pantoea agglomerans and culturing the Pantoea agglomerans is combined.

4. A plant disease resistance inducer, wherein a fermented culture obtained by fermenting a plant with Pantoea agglomerans and culturing the Pantoea agglomerans is combined.

5. A method of controlling a disease of a plant, wherein the disease of the plant is controlled by the plant disease resistance inducer according to claim 2 or 4.
